# EUROPEAN PATENT SPECIFICATION

(11) **EP 4 078 170 B1**
(45) Date of publication and mention of the grant of the patent: **27.05.2026**
(21) Application number: 19839335.7
(22) Date of filing: 20.12.2019
(51) Int. Cl.: G01N 31/22, G01N 21/77

(54) **CHEMO SENSOR WITH SULPHUR-CONTAINING IONOPHORES**
CHEMISCHER SENSOR MIT SCHWEFEL-ENTHALTENDEN IONOPHOREN
CAPTEUR CHIMIQUE AVEC IONOPHORES CONTENANT DU SOUFRE

(43) Date of publication of application: 26.10.2022
(73) Proprietor: Robert Bosch GmbH, 70442 Stuttgart (DE)
(72) Inventor: PALALE, Suresh, Singapore 820648 (SG); SAGAR, Kaushal, Singapore 787082 (SG)
(86) International application number: PCT/EP2019/086508
(87) International publication number: WO 2021/121613

(56) References cited:
- CN-A- 1 752 750
- US-A1- 2008 044 913
- US-A1- 2010 041 158
- KRAITHONG SASIWIMON ET AL: "A method to detect Hg2+ in vegetable via a "Turn-ON" Hg2+-Fluorescent sensor with a nanomolar sensitivity", JOURNAL OF PHOTOCHEMISTRY AND PHOTOBIOLOGY, A: CHEMISTRY, ELSEVIER SEQUOIA, LAUSANNE, CH, vol. 389, 9 November 2019 (2019-11-09), XP085964360, ISSN: 1010-6030, [retrieved on 20191109], DOI: 10.1016/J.JPHOTOCHEM.2019.112224

## Description

### Technical Field

The present invention generally relates to a sulphur-containing compound as chemosensor for lead ion detection in water, a method for detecting lead ions and use of the chemosensor for fluorescence measurement of lead ions.

### Background

Heavy metal pollution in drinking water is a prevailing problem, particularly in the Asian region. The common drinking water sources such as lakes or rivers are polluted directly and indirectly with industrial and consumer waste materials of different kinds. Lead pollution is one of the highest heavy metal pollution in water and long term exposure of lead to human beings can result in grave health issues. Therefore, it is required to ensure drinking water is free from lead ions. Presently available techniques (ICP-MS, ICP- atomic emission spectroscopy, flame atomic absorption spectroscopy, anodic stripping voltammetry, DNA based sensors) are all batch processes; both lab and point of care based. There is a need for new chemosensors to improve lead detection in water, which should be ultra-sensitive for lead ion detection in accordance with WHO guidelines (ppb or ug/L range). Additionally, they should provide for a high selectivity for lead ion detection in water. Ideally, there should be no pH cross sensitivity of the chemosensors. Furthermore, they should provide for real-time measurement and be self-regenerating for monitoring lead ion in drinking water.

US 2010/041158 A1 discloses a sensor for detecting lead in an aqueous sample.

Kraithong Sasiwimon et al., "a method to detect Hg2+ in vegetable via a "Turn-ON" Hg2+-Fluorescent sensor with a nanomolar sensitivity", Journal of Photochem Photobiology, A: Chemistry vol. 389, 9 November 2019, discloses a Hg²⁺ colorimetric and fluorescent sensor.

CN 1 752 750 A discloses a sensor compound based on rhodamine 6G and a pmethoxyphenyl thiocarbamide.

US 2008/044913 A1 discloses a compound for mercury-ion selective detection.

In view of the above, there is a need for a chemosensor which can overcome, or at least ameliorate, some of the problems discussed above.

### Summary

In a first aspect, the present invention provides a chemosensor for lead ion detection in water comprising a sensor layer placed in an optoelectrical transducer. The sensor layer comprises at least one compound having at least one sulphur-containing ionophore moiety and a fluorophore moiety, wherein the at least one compound having the at least one sulphur-containing ionophore moiety and the fluorophore moiety is a compound of one of the following Formulae (II), (III) 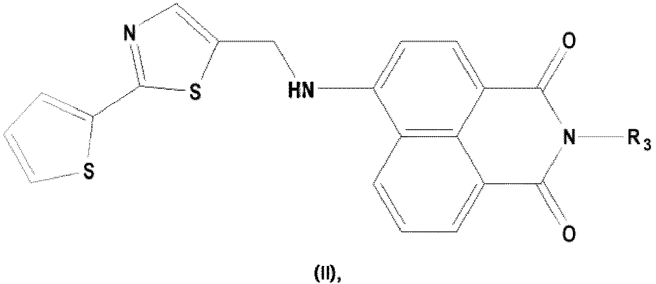 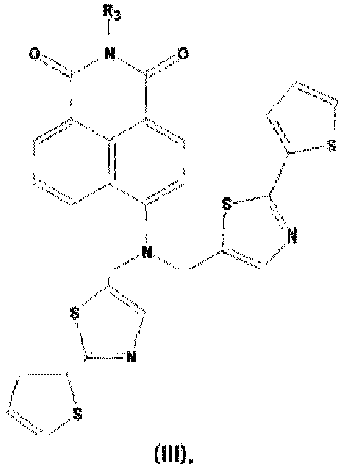 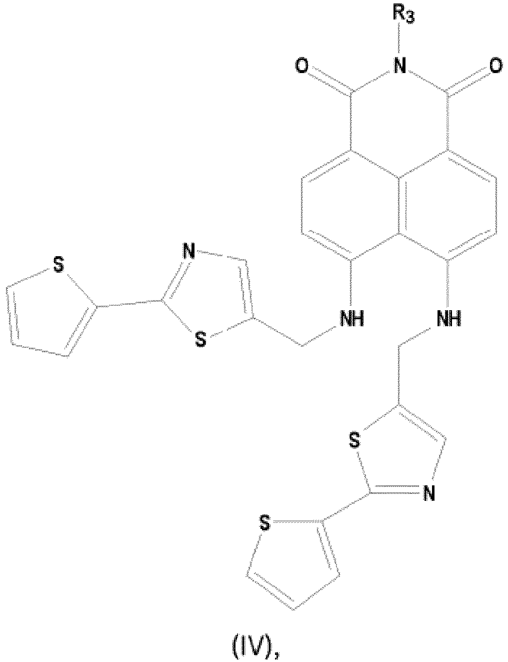 wherein R3 is hydrogen or an organic moiety.

In a second aspect, the present invention provides a method of detecting lead ions in a water sample, the method comprising i) providing a chemosensor as described in various embodiments of the first aspect for lead ion detection in water; ii) measuring the fluorescence signal emitted from the sensor layer; and iii) attributing the fluorescence signal to a lead ion concentration in the water.

In a third aspect, the present invention provides the use of a chemosensor as described in various embodiments of the first aspect mentioned above for lead ion detection in water.

### Brief Description of the Drawings

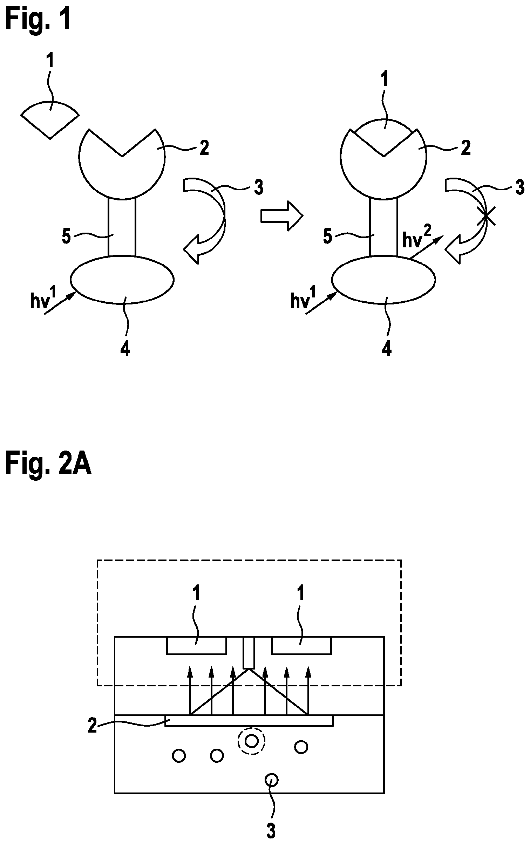

FIG. 1 illustrates the basic principle of fluorescent chemosensor technology on which the present disclosure is based on. 1 refers to a lead ion, 2 refers to a sulphur-containing ionophore moiety, 3 refers to the photo-induced electron transfer (PET), 4 refers to the fluorophore moiety, and 5 refers to an optional spacer. The sensor molecule comprises two main components: receptor or ionophore moiety and fluorophore moiety. The fluorophore is a fluorescent moiety which exhibits a particular excitation wavelength and upon excitation (signified as hυ¹) emits fluorescence signal at a higher wavelength. The receptor moiety, or ionophore moiety, is very specific in terms of chelating/binding to a guest molecule or an analyte, such as lead ions. An empty receptor site would result in quenching of the fluorescence signal due to photo-induced electron transfer (PET) of an electron from electron rich receptor moiety to the fluorescent moiety. In the event of a lead ion chelating with the ionophore moiety, the PET effect is nullified, as no electron can undergo the PET, resulting in enhanced fluorescence signal (signified as hυ²). The signal strength is in proportion to the lead ion concentration and thus can be used for quantitative analysis of the lead ion.
FIG. 2A illustrates a schematic diagram of an optoelectronic transduction system for fluorescence based chemo sensing, wherein 1 refers to an optical analysis module, 2 refers to a sensor layer, and 3 refers to lead ions.

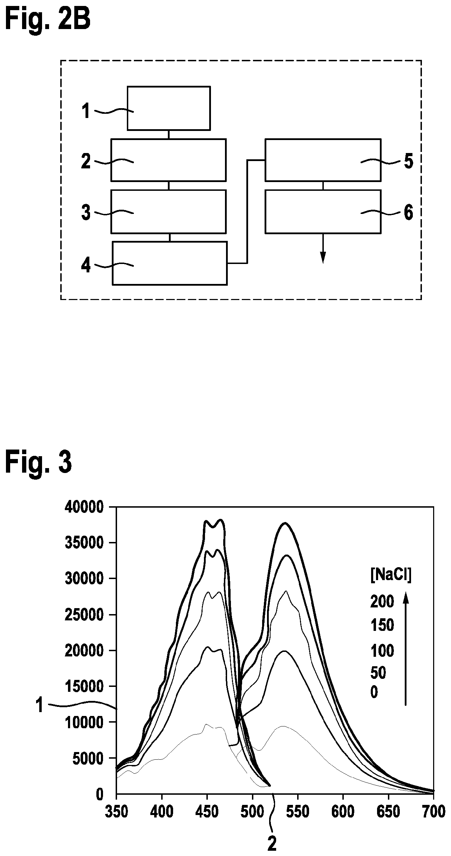

FIG. 2B illustrates a schematic diagram of an optoelectronic transduction system for fluorescence based chemo sensing, wherein 1 refers to a light-emitting diode (LED), optionally emitting at a wavelength of 430 to 470 nm, 2 refers to an optical filter, optionally with a narrow band pass, optionally being at 430 nm/40 nm, 3 refers to a colored glass filter, optionally with a long pass, optionally from 530 nm onwards, 4 refers to an optical analyzer, optionally a photo diode, and 5 refers to a microprocessor, and 6 refers to a software interface.
FIG. 3 shows a graph representing a sample fluorescence excitation (on left) and emmission peak (on right) for sodium ion based chemosensing. This graph is typically encountered in the prior art. The captions on the graph are numbered 1 and 2, wherein 1 refers to "Fluorescence Intensity" and 2 refers to "Wavelength (nm)".

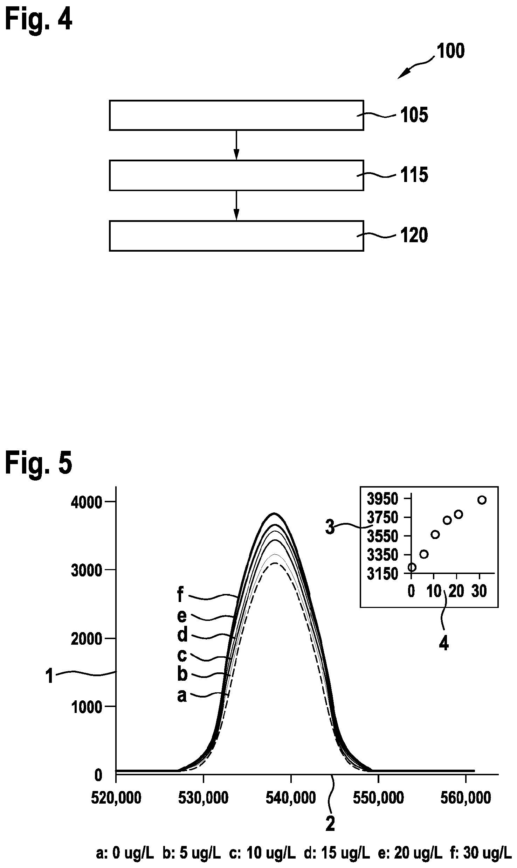

FIG. 4 is a process flow chart describing the individual method steps of the method 100, defined as follows: In step 105, a chemosensor for lead ion detection in water is provided, comprising a sensor layer placed in an optoelectrical transducer, the sensor layer comprising at least one compound having at least one sulphur-containing ionophore moiety and a fluorophore moiety. In step 115, the fluorescence signal emitted from the sensor layer is measured. In step 120, the fluorescence signal is attributed to a lead ion concentration in the water.
FIG. 5 shows the emission fluorescence spectra using a compound having at least one sulphur ionophore moiety and a naphthalimide fluorophore moiety during detection of lead ion in water. The captions on the graph which are numbered 1 and 2 refer to 1: "Fluorescence Intensity, a.u." and 2: "Wavelength, nm". In the inlet, the captions on the graph which are numbered 3 and 4 refer to 1: "Intensity" and 4: "Concentration". The letters a, b, c, d, e and f refer to the difference in concentration, wherein "a" refers to the lowest curve while the highest concentration "f" refers to the highest curve.

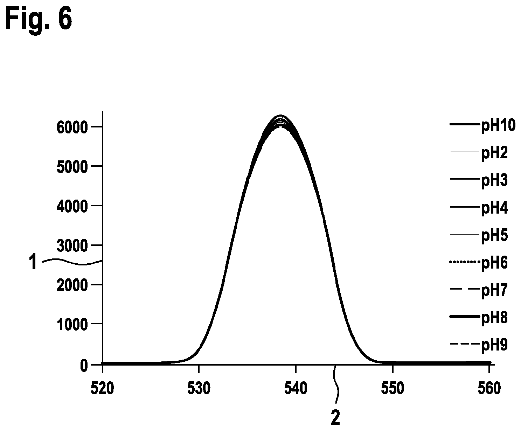

FIG. 6 shows a graph illustrating the pH insensitivity of a compound having at least one sulphur-containing ionophore moiety and a naphthalimide fluorophore moiety during detection of 10ug/L lead ion solution in water. The captions on the graph which are numbered 1 and 2 refer to 1: "Fluorescence Intensity, a.u." and 2: "Wavelength, nm". Since the curves are substantially overlapping, it can be concluded that the fluorescence intensity is independent of the pH value.

### Detailed Description

The improvement of the presently presented chemosensor may result from the selection of the ionophore moiety. Most of the prior art relates to chemosensor technology leverages on the combination of nitrogen and oxygen based ionophore moieties for the formation of the metal chelating ring structure. The electron rich nitrogen in the prior art is utilized in both the photo-induced electron transfer (PET) and coordination with metal ion. Herein, it is proposed that sensor molecules with sulphur-based ionophore moieties are utilized, replacing nitrogen. This is because sulphur-based ionophore moieties have a higher binding affinity towards lead ion. Furthermore, they are specific for chelation with lead ions as well. Finally, unlike nitrogen, sulphur-based ionophore moieties do not take part in pH titration reactions, making the sensor layer insensitive to pH changes which may come from the water sample.

Accordingly, in one aspect, there is provided a chemosensor for lead ion detection in water comprising a sensor layer placed in an optoelectrical transducer, the sensor layer comprising at least one compound having at least one sulphur-containing ionophore moiety and a fluorophore moiety.

According to various embodiments, the at least one sulphur-containing ionophore moiety may comprise a functional group selected from the group consisting of thiourea, thioamide, thioether, thiophene, thiazole, thiomorpholine, -NH-C(S)-NH-NH₂, and a combination thereof.

According to various embodiments, the at least one sulphur-containing ionophore moiety may have a high binding efficiency towards lead ions to be used as receptor sites. Hence, the at least one sulphur-containing ionophore moiety may be selected from the group consisting of 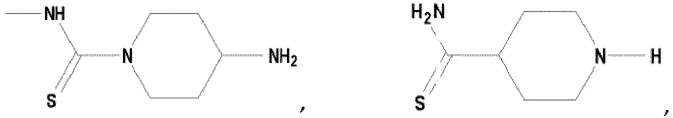 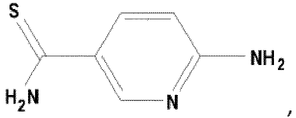 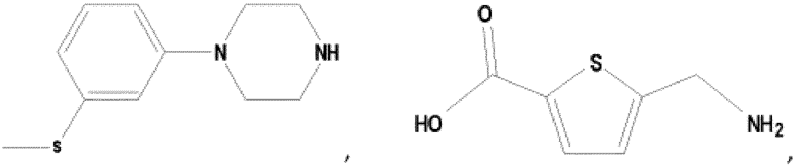 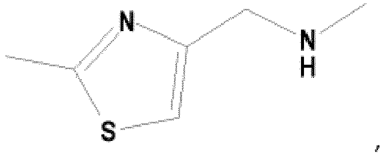 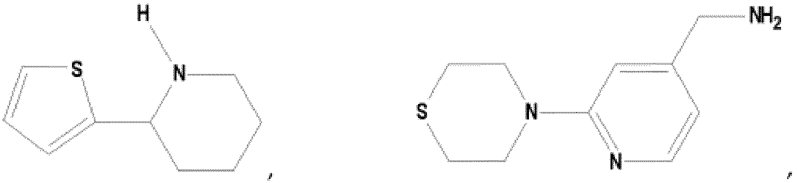 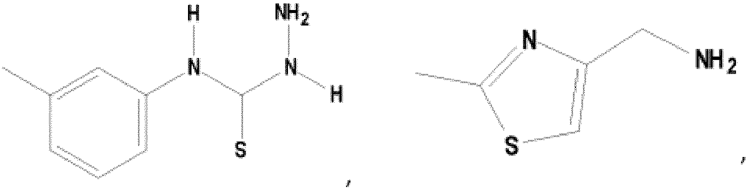 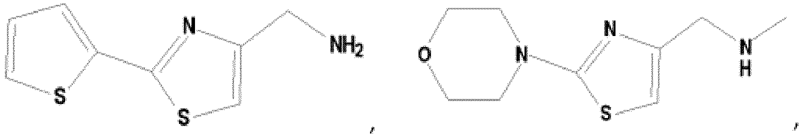 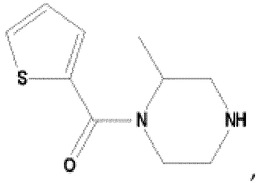 and a combination thereof.

According to various embodiments, the at least one compound having at least one sulphur-containing ionophore moiety and a fluorophore moiety is a compound of the following Formula (I) 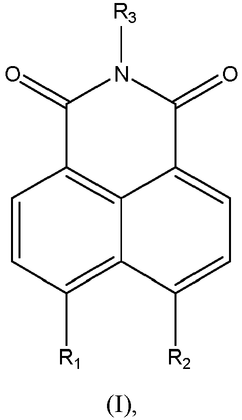 wherein:
R₁ comprises a functional group selected from the group consisting of thiourea, thioamide, thioether, thiophene, thiazole, thiomorpholine, -NH-C(S)-NH-NH₂, and a combination thereof;
R₂ is selected from the group consisting of hydrogen, an unsubstituted, linear or branched C₁-C₂₀-alkyl and R₁; and
R₃ is hydrogen or an organic moiety.

In various embodiments, R₁ may be a secondary amine, wherein the nitrogen atom may resemble the point of contact to any one of the sulphur-containing ionophore moieties described above. Alternatively, R₁ may be a tertiary amine, wherein the nitrogen atom may resemble the point of contact to any two of the sulphur-containing ionophore moieties described above (selected individually).

In various embodiments, R₂ may be defined as R₁, whereby it is understood that in each instance R₁ is individually selected from the embodiments above. Said differently, when R₂ is R₁, both moieties R₁ pendant on the fluorophore moiety may represent an ionophore selected individually from the embodiments associated with R₁ as detailed above.

With regard to R₃, the term 'an organic moiety' as used herein refers to carbon-containing moieties. These moieties can be linear or branched, substituted or unsubstituted, and are derived from hydrocarbons, typically by substitution of one or more carbon atoms by other atoms, such as oxygen, nitrogen, sulfur, phosphorous, or functional groups that contain oxygen, nitrogen, sulphur, phosphorous. Suitable groups and moieties are well known to those skilled in the art or can be readily identified by routine experimentation.

In a preferred embodiment, the organic moiety can be a linear or branched, substituted or unsubstituted alkyl with 1 to x carbon atoms; linear or branched, substituted or unsubstituted alkenyl with 2 to x carbon atoms; linear or branched, substituted or unsubstituted alkynyl with 2 to x carbon atoms; linear or branched, substituted or unsubstituted alkoxy with 1 to x carbon atoms; substituted or unsubstituted cycloalkyl with 3 to x carbon atoms; substituted or unsubstituted cycloalkenyl with 3 to x carbon atoms; substituted or unsubstituted aryl with 6 to x carbon atoms; and substituted or unsubstituted heteroaryl with 3 to x carbon atoms; with x being any integer of 2 or more.

In a further embodiment of the present disclosure, the organic moiety can be a linear or branched, substituted or unsubstituted alkyl with 1 to 10 carbon atoms; linear or branched, substituted or unsubstituted alkenyl with 3 to 10 carbon atoms; linear or branched, substituted or unsubstituted alkoxy with 1 to 10 carbon atoms, substituted or unsubstituted cycloalkyl with 5 to 10 carbon atoms; substituted or unsubstituted cycloalkenyl with 5 to 10 carbon atoms; substituted or unsubstituted aryl with 5 to 10 carbon atoms; and substituted or unsubstituted heteroaryl with 5 to 10 carbon atoms.

The organic moiety can also be a combination of any of the above-defined groups, including but not limited to alkylaryl, arylalkyl, alkyl heteroaryl and the like, to name only a few, all of which may be substituted or unsubstituted.

The term "substituted" as used herein in relation to the above moieties refers to a substituent other than hydrogen. Such a substitution is preferably selected from the group consisting of halogen, -OH, -OOH, -NH₂, -NO₂, -ONO₂, -CHO, -CN, -CNOH, -COOH, -SH, - OSH, -CSSH, -SCN, -SO₂OH, -CONH₂, -NH-NH₂, -NC, -CSH -OR, -NRR', -NR, -C(O)R, -C(O)OR, - (CO)NRR', -NR'C(O)R, -OC(O)R, aryl with 5 to 10 carbon atoms, cycloalk(en)yl with 3 to 10 carbon atoms, 3- to 8-membered heterocycloalk(en)yl, and 5-to 10-membered heteroaryl, wherein R and R' are independently selected from hydrogen, alkyl with 1 to 10 carbon atoms, alkenyl with 2 to 10 carbon atoms, alkynyl with 2 to 10 carbon atoms, aryl with 5 to 10 carbon atoms, cycloalk(en)yl with 3 to 10 carbon atoms, 5- to 10- membered heteroaryl, comprising 1 to 4 heteroatoms selected from nitrogen, oxygen, and sulfur, and 5- to 10- membered heterocycloalk(en)yl, comprising 1 to 4 heteroatoms selected from nitrogen, oxygen, and sulfur. Any of these substituents may again be substituted, it is however preferred that these substituents are unsubstituted.

Alkyl, or abbreviated 'alk', refers to a saturated hydrocarbon moiety, such as methyl, ethyl, and the like. Alkenyl and alkynyl comprise at least one carbon-carbon double bonds or triple bonds, respectively, and are otherwise defined as alkyl above.

Cycloalkyl refers to a non-aromatic carbocyclic moiety, such as cyclopentanyl, cyclohexanyl, and the like.

Cycloalkenyl refers to non-aromatic carbocyclic compounds that comprise at least one C-C double bond.

Similarly, heterocycloalk(en)yl relates to cycloalk(en)yl groups wherein 1 or more ring carbon atoms are replaced by heteroatoms, preferably selected from nitrogen, oxygen, and sulfur.

Aryl relates to an aromatic ring that is preferably monocyclic. Preferred aryl substituents are moieties with 6 carbon atoms, such as phenyl.

Heteroaryl refers to aromatic moieties that correspond to the respective aryl moiety wherein one or more ring carbon atoms have been replaced by heteroatoms, such as nitrogen, oxygen, and sulfur.

All of the afore-mentioned groups can be substituted or unsubstituted. When substituted, the substituent can be selected from the above list of substituents.

The term 'at least one' as used herein relates to one or more, for example 1, 2, 3, 4, 5, 6, 7, 8, 9 or more of the referenced species.

Halogen as used herein refers to F, CI, Br, and I.

In various embodiments, the compound having at least one sulphur-containing ionophore moiety and a fluorophore moiety may be a compound of one of the following Formulae (II), (III) or (IV) 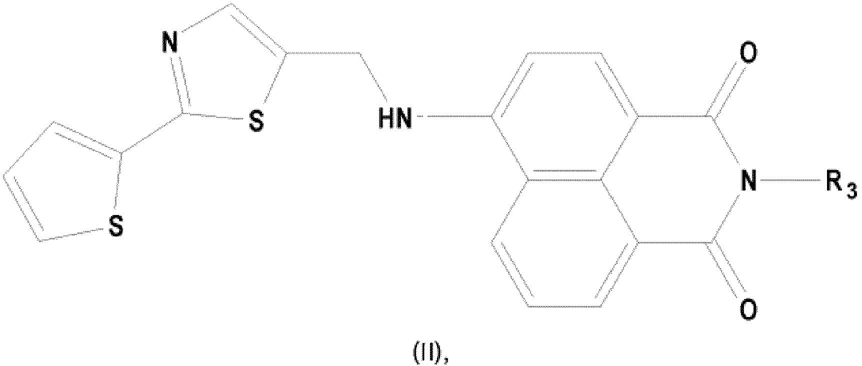 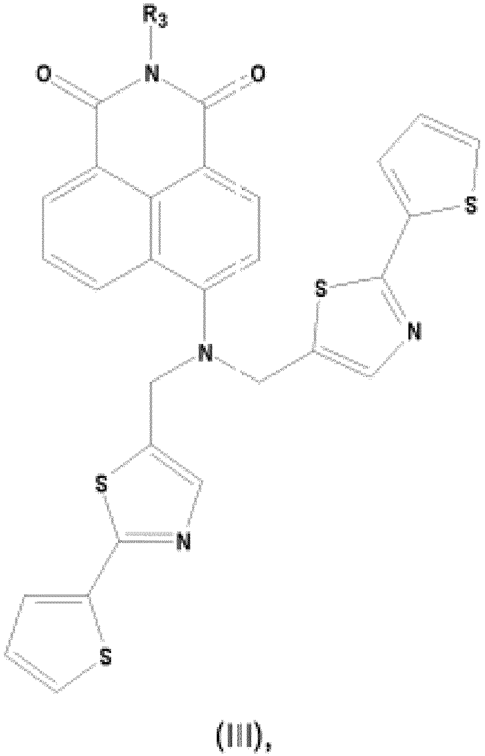 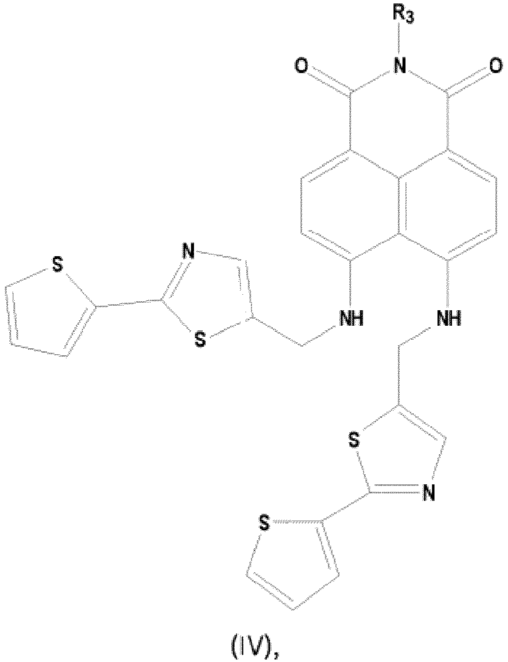 wherein R₃ is hydrogen or an organic moiety as defined above.

According to various embodiments, the fluorophore moiety may be a non-interfering photo stable fluorophore with larger stokes shift. It may therefore be suitable to be used to covalently bind with the receptor moiety (otherwise termed herein as the sulphur-containing ionophore moiety). The fluorophore moiety may be selected from the group consisting of 1,2-bis(2-aminophenoxy) ethane-N,N,N',N'-tetraacetic acid (BAPTA), 2-(2'-morpholino-2'-oxoethoxy)-N,N-bis(hydroxycarbonylmethyl) aniline (MOBHA), fluorescein, rhodamine, naphthalimide, and a combination thereof.

According to various embodiments, the water wherein the lead ion is to be detected is drinking water, sea water, pool water, river water, lake water, aqua culture, waste water, and a combination thereof.

According to various embodiments, the substrate on which the sensor layer is attached to is a hydrophilic or hydrophobic polymer, glass, silicon, natural fiber, and a combination thereof. The substrate may be physically or chemically attached to the sensor layer.

According to various embodiments, the optoelectrical transducer comprises a light-emitting diode (LED), from which an optical pathway passes an optical filter, the sensor layer, and subsequently a colored glass filter and a photo diode.

Optical sensing typically requires complicated optoelectronic circuitry for sensing purposes. This may include filters, beam collimators, optical fibre, laser, spectrometer etc. These components make the sensor transduction less lucrative from a cost, durability and difficulty in miniaturization point of view. Herein, an approach of sensor transduction is proposed where the optoelectronic system may remain lean and cost-effective by excluding many of the routine components, which would otherwise have been essential for traditional circuitry. The main improvement may be the result from the arrangement of the system components. This may include an LED source instead of a laser, a mechanical design to exclude collimators, colored glass instead of optical filters and the utilization of a photodiode instead of spectrometer. This system may change a traditional optical transduction system into a leaner and effective measurement system. The objective of this embodiment is to measure fluorescence change coming from the sensor layer, which may be proportionate to the binding event of lead ion on the chemosensor layer. The change of fluorescence emission (output) would only be expected in its amplitude and not in shift of wavelength. A prior art embodiment wherein the change of fluorescence emission is measured in shift of wavelength is depicted in FIG. 3. As this shift of wavelength is not expected in the present disclosure, the present mechanism presents an opportunity to focus only on emission peak wavelength region, controlled by optical filtering through colored glass and thus converting fluorescence photon energy into an electrical signal using a photo diode. This mechanism may eliminate the need of having a spectrometer, which is an expensive and bulky component. Further with the help of standard electronic circuitry, the electrical signal may be amplified and processed through microprocessor and software to display final lead ion concentration in water stream.

In a second aspect, there is provided a method (100) of detecting lead ions in a water sample, the method comprising
i) providing a chemosensor as described in various embodiments of the first aspect;
ii) measuring a fluorescence signal emitted from the sensor layer (115); and
iii) attributing the fluorescence signal to a lead ion concentration in the water (120).

According to various embodiments, step ii) may comprise measuring the fluorescence signal emitted from the sensor layer with a photo diode, whereby a change in amplitude of the fluorescence signal is measured.

In a third aspect, there is provided use of a chemosensor as described in various embodiments of the first aspect for lead ion detection in water.

### Examples

### Example 1: Emission fluorescence spectra of using a compound having at least one sulphur ionophore moiety and a naphthalimide fluorophore moiety during detection of lead ion in water

Lead nitrate solutions are prepared in deionized (DI) water at room temperature. Concentration of free lead ions in water is in the range of 0-30 ug/L. No sensor regeneration or pre or posttreatment of sensor layer was carried out between the measurements, indicating that it is real-time sensing capability. The results are shown in FIG. 5. As may be seen, the intensity is proportional to the concentration of lead ions, thereby enabling in a detectable and reliable lead ion measurement.

### Example 2: pH insensitivity of a compound having at least one sulphur-containing ionophore moiety and a naphthalimide fluorophore moiety during detection of 10ug/L lead ion solution in water

In this Example, the pH sensitivity of sulphur-containing ionophore moiety and naphthalimide fluorophore moiety chemosensor at 10ug/L lead ion solution in water is measured. The result is shown in FIG. 6. As may be seen, the measurement is nearly constant across the pH range, showing that the disclosed chemosensor is not sensitive (or only in negligible amount) to a pH value in the water sample.

## Claims

1. A chemosensor for lead ion detection in water comprising a sensor layer placed in an optoelectrical transducer, the sensor layer comprising at least one compound having at least one sulphur-containing ionophore moiety and a fluorophore moiety, wherein the at least one compound having at least one sulphur-containing ionophore moiety and a fluorophore moiety is a compound of one of the following Formulae (II), (III) or (IV) 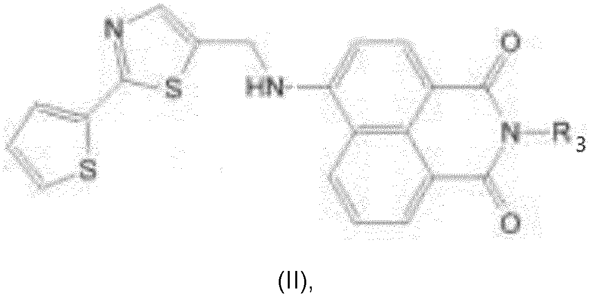 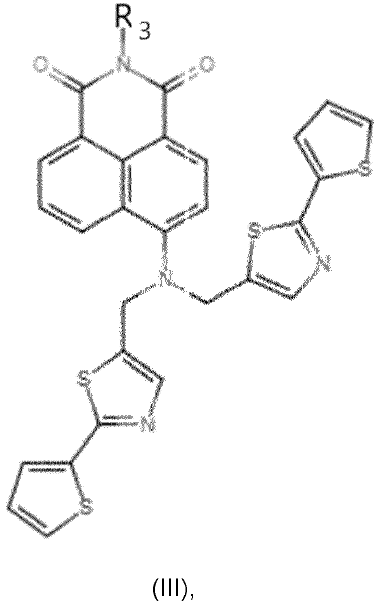 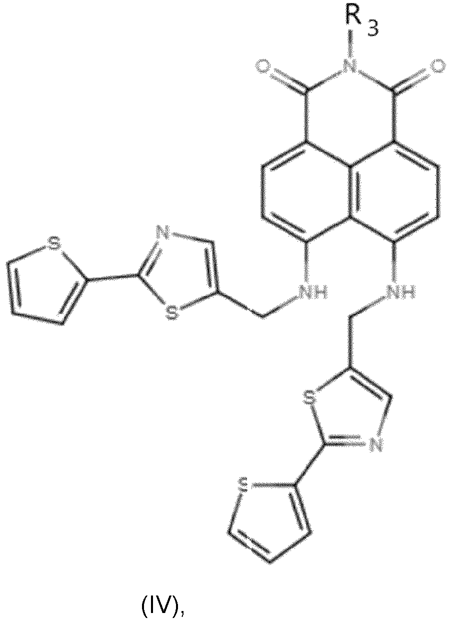 wherein R₃ is hydrogen or an organic moiety.

2. The chemosensor of claim 1, wherein the lead ion is to be detected is drinking water, sea water, pool water, river water, lake water, aqua culture, waste water, and a combination thereof.

3. The chemosensor of any one of the preceding claims, wherein the optoelectrical transducer comprises a light-emitting diode (LED), from which an optical pathway passes an optical filter, the sensor layer, and subsequently a colored glass filter and a photo diode.

4. A method (100) of detecting lead ions in a water sample, the method comprising
i) providing a chemosensor of any one of claims 1 to 3 for lead ion detection in water;
ii) measuring a fluorescence signal emitted from the sensor layer (115); and
iii) attributing the fluorescence signal to a lead ion concentration in the water (120).

5. The method (100) of claim 4, wherein step ii) comprises measuring the fluorescence signal emitted from the sensor layer with a photo diode, whereby a change in amplitude of the fluorescence signal is measured.

6. Use of a chemosensor of any one of claims 1 to 3 for lead ion detection in water.

## Patentansprüche

1. Chemosensor zur Bleiionendetektion in Wasser, umfassend eine Sensorschicht, die in einem optoelektrischen Wandler platziert ist, wobei die Sensorschicht mindestens eine Verbindung mit mindestens einem schwefelhaltigen lonophoranteil und einem Fluorophoranteil umfasst, wobei die mindestens eine Verbindung mit mindestens einem schwefelhaltigen lonophoranteil und einem Fluorophoranteil eine Verbindung von einer der folgenden Formeln (II), (III) oder (IV) ist: 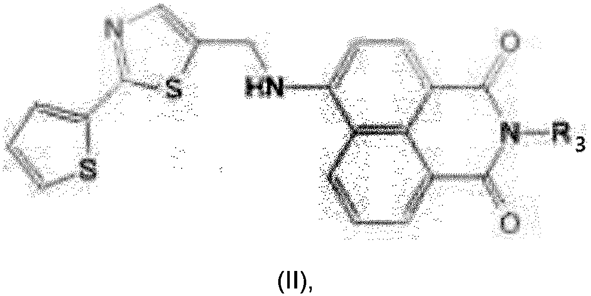 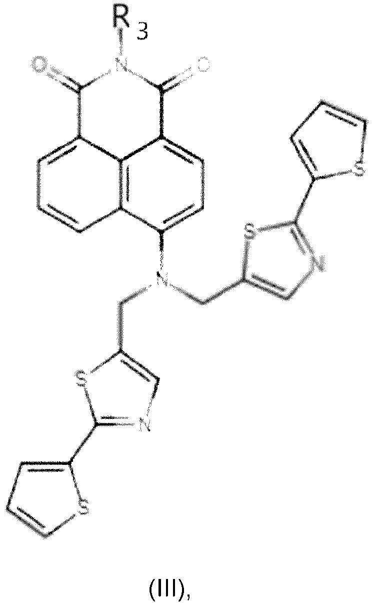 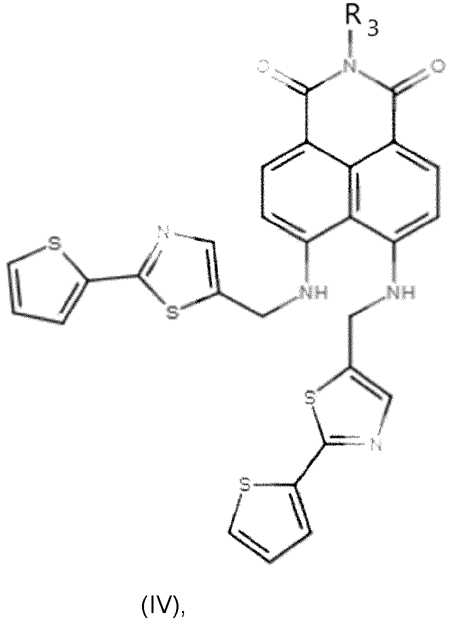 wobei R₃ Wasserstoff oder ein organischer Anteil ist.

2. Chemosensor nach Anspruch 1, wobei das Bleiion in Trinkwasser, Meerwasser, Poolwasser, Flusswasser, Seewasser, Aquakultur, Abwasser und einer Kombination davon zu detektieren ist.

3. Chemosensor nach einem der vorhergehenden Ansprüche, wobei der optoelektrische Wandler eine Leuchtdiode (LED) umfasst, von der ein optischer Pfad ein optisches Filter, die Sensorschicht und anschließend ein Farbglasfilter und eine Fotodiode passiert.

4. Verfahren (100) zum Detektieren von Bleiionen in einer Wasserprobe, wobei das Verfahren umfasst:
i) Bereitstellen eines Chemosensors nach einem der Ansprüche 1 bis 3 zur Bleiionendetektion in Wasser;
ii) Messen eines Fluoreszenzsignals, das von der Sensorschicht (115) emittiert wird; und
iii) Zuordnen des Fluoreszenzsignals zu einer Bleiionenkonzentration im Wasser (120).

5. Verfahren (100) nach Anspruch 4, wobei Schritt ii) Messen des von der Sensorschicht emittierten Fluoreszenzsignals mit einer Fotodiode umfasst, wodurch eine Änderung der Amplitude des Fluoreszenzsignals gemessen wird.

6. Verwendung eines Chemosensors nach einem der Ansprüche 1 bis 3 zur Bleiionendetektion in Wasser.

## Revendications

1. Chimiodétecteur pour la détection d'ions plomb dans l'eau comprenant une couche de détection placée dans un transducteur optoélectrique, la couche de détection comprenant au moins un composé ayant au moins un fragment ionophore contenant du soufre et un fragment fluorophore, l'au moins un composé ayant au moins un fragment ionophore contenant du soufre et un fragment fluorophore étant un composé répondant à l'une des formules (II), (III) ou (IV) suivantes 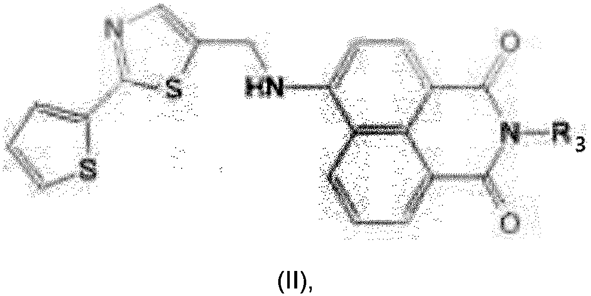 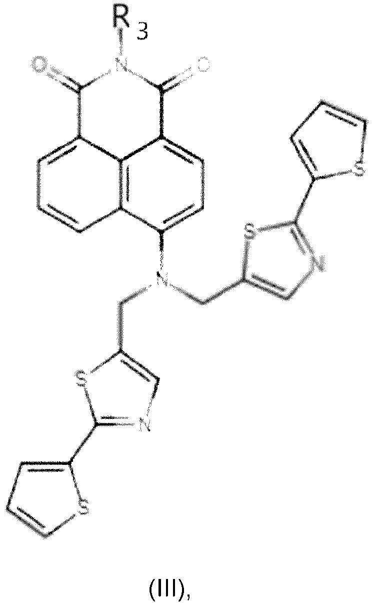 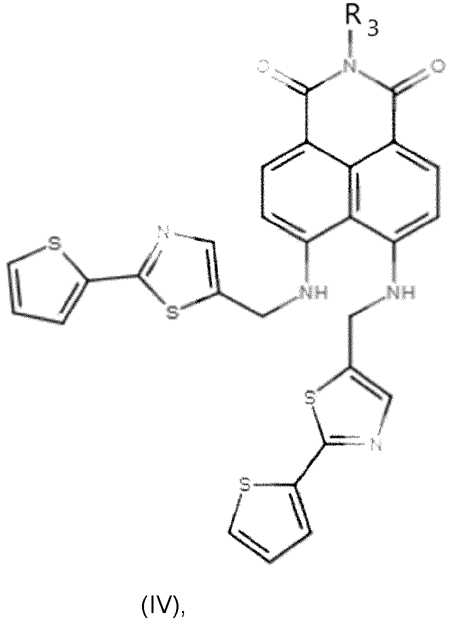 R₃ étant un hydrogène ou un fragment organique.

2. Chimiodétecteur selon la revendication 1, l'ion plomb devant être détecté dans l'eau potable, l'eau de mer, l'eau de piscine, l'eau de rivière, l'eau de lac, la culture aquatique, les eaux usées et une combinaison de celles-ci.

3. Chimiodétecteur selon l'une quelconque des revendications précédentes, le transducteur optoélectrique comprenant une diode électroluminescente (DEL), à partir de laquelle un trajet optique passe par un filtre optique, la couche de détection, et ensuite un filtre en verre coloré et une photodiode.

4. Procédé (100) de détection d'ions plomb dans un échantillon d'eau, le procédé comprenant
i) la fourniture d'un chimiodétecteur selon l'une quelconque des revendications 1 à 3 pour une détection des ions plomb dans l'eau ;
ii) la mesure d'un signal de fluorescence émis à partir de la couche de détection (115) ; et
iii) l'attribution du signal de fluorescence à une concentration d'ions plomb dans l'eau (120).

5. Procédé (100) selon la revendication 4, l'étape ii) comprenant la mesure du signal de fluorescence émis par la couche de détection avec une photodiode, moyennant quoi un changement d'amplitude du signal de fluorescence est mesuré.

6. Utilisation d'un chimiodétecteur selon l'une quelconque des revendications 1 à 3 pour la détection d'ions plomb dans l'eau.
